Europäisches Patentamt

European Patent Office

Office européen des brevets

(11) Publication number: **0 330 151 A2**

# EUROPEAN PATENT APPLICATION

(21) Application number: 89103012.4

(22) Date of filing: 21.02.89

(51) Int. Cl.⁴: **C08L 53/00 , C08L 23/12 , B65D 30/02 , A61J 1/00 , //(C08L53/00,23:12,23:08), (C08L23/12,23:08,53:00)**

The title of the invention has been amended (Guidelines for Examination in the EPO, A-III, 7.3).

(30) Priority: 23.02.88 JP 40200/88

(43) Date of publication of application:
30.08.89 Bulletin 89/35

(84) Designated Contracting States:
BE DE ES FR GB NL SE

(71) Applicant: **NISSHO CORPORATION**
**9-3, Honjo-nishi, 3-chome Oyodo-ku Osaka-shi(JP)**

(72) Inventor: **Hitoshi, Futagawa**
**4-15, Marunouchi-cho Otsu-shi Shiga-ken(JP)**

(74) Representative: **Türk, Gille, Hrabal Bruckner Strasse 20 D-4000 Düsseldorf 13(DE)**

(54) **Bag for the storage of blood platelets.**

(57) A bag for storage of platelets comprising a polymer alloy consisting essentially of (a) 25 to 60 % by weight of poly(ethylene-butylene)-polystyrene block copolymer, (b) 20 to 40 % by weight of polypropylene, and (c) 5 to 35 % by weight of ethylene-acrylic acid ester copolymer, the segment of said polystyrene in the molecular chains of said polymer (a) having at least 2 oriented portions and the molecular chains of the polymers (a), (b) and (c) being entangled to form a network structure, and a multiple bag system comprising a donor bag, a transfer bag and tubes for connecting the donor bag with the transfer bag. The bag and the multiple bag system have excellent chemical stability and show excellent mechanical strength at high or low temperatures.

FIG. 1

## BAG FOR STORAGE OF PLATELETS

The present invention relates to a bag for storage of platelets and a multiple bag system comprising the bags, and more particularly to a bag for storage of platelets and a multiple bag system in which the blood platelet can be stored for a long period of time since the blood platelet does not adhere onto the inner surface of the bags and chemical substances do not elute from the inner surface of the bags when the blood platelet is stored in the bags.

As a bag for storage of platelets which can be safely stored for a long period of time and can be supplied to a patient in case of necessity, a bag made of polyvinyl chloride containing a plasticizer such as dioctyl phthalate has hitherto been well-known.

However, the bag has the defects that the bag is poor in gas permeability, that blood platelet is easily adhered onto the surface of the bag when the blood platelet is stored in the bag, and that aggregation property of the blood platelet has already been lowered when the blood platelet is supplied to a patient. Also, the bag has a defect that there is a difficulty to charge the blood platelet into the bag because the bag shows blocking property at the time of being subjected to high-pressure steam sterilization and the inner surfaces of the bag are easily adhered together. Further, the bag has another defect that the functions of the blood platelet such as hypotonic shock response deteriorate when the blood platelet is stored in the bag because a plasticizer being contained in polyvinyl chloride which composes the bag elute into blood platelet during the blood platelet is stored in the bag for a long period of time.

As a bag which removes the above-mentioned defects, there is proposed a bag made of polymer composition prepared by mixing (A) poly(ethylene- butylene)-polystyrene block copolymer with (B) polypropylene and (C) ethylene-vinyl acetate copolymer or polyethylene as disclosed in U.S. Pat. No. 4,140,162.

The bag is also used in a multiple bag system which comprises a donor bag for receiving blood from a donor and at least one transfer bag for storing blood platelet separated from the blood which is stored in the donor bag. An example of the multiple bag system which is disclosed in U.S. Pat. No. 4,222,379 is well-known. The transfer bag used in the multiple bag system is made of the polymer composition disclosed in the above-mentioned U.S. Pat. No. 4,140,162.

The transfer bag used herein has an advantage that additives such as a plasticizer contained in the bag do not elute into the blood platelet because additives are not contained in the bag. However, the bag has disadvantages that the bag is softened and transformed when the bag is subjected to high-pressure steam sterilization because elastic modulus of the material of the bag is lowered at high temperatures. Particularly, the bag made of a polymer mixture, in which ethylene-vinyl acetate copolymer is contained, has a defect that acetic acid, which is generated from the bag as the results of heat decomposition at the time of forming, or hydrolysis at the time of high-pressure steam sterilization, is dissolved into a concentrated solution of blood platelet so that the concentrated solution is acidified.

The present invention has been studied to give a bag for storage of platelets from which the above-mentioned disadvantages are removed. As the results of researches, when blood platelet is stored in a bag for storage of platelets made of polymer alloy prepared by mixing poly(ethylene-butylene)-polystyrene block copolymer and polypropylene with ethylene-acrylic acid ester copolymer, it has been eventually found that no chemical substances elute from the bag into the blood platelet, that the bag is not deformed at high or low temperatures, and that the bag has high gas permeability, therefore the bag can be used as a bag for storage of platelets for a long period of time.

The present invention has been accomplished in accordance with the above-mentioned results.

These and other objects of the present invention will become apparent from the description hereinafter.

In accordance with the present invention, there is provided a bag for storage of platelets comprising polymer alloy consisting essentially of

(a) 25 to 60 % by weight of poly(ethylene-butylene)-polystyrene block copolymer,

(b) 20 to 40 % by weight of polypropylene, and

(c) 5 to 35 % by weight of ethylene-acrylic acid ester copolymer, the segment of said polystyrene in the molecular chains of said polymer (a) having at least 2 oriented portions and the molecular chains of said polymers (a), (b) and (c) being entangled to form a network structure.

In accordance with the present invention, there is also provided a multiple bag comprising a donor bag for receiving blood from a donor and for separating the blood components, one or more than one transfer bags for storing the separated blood components and tubes for transferring the separated blood components from the donor bag to the transfer bag, said transfer bag comprising said polymer alloy.

2

In accordance with the present invention, there is also provided a multiple bag of which the donor bag comprises polyvinyl chloride containing dioctyl phthalate.

In the present invention, a polymer alloy is prepared by mixing ethylene-acrylic acid ester copolymer with a mixture of poly(ethylene-butylene)-polystyrene block copolymer and polypropylene in a specific ratio, due to the soluble interaction of the ethylene-acrylic acid ester copolymer which gives compatibility the poly(ethylene-butylene)-polystyrene block copolymer and polypropylene.

Also, a bag made of the polymer alloy is preferably used as a bag for storage of platelets since the blood platelet does not adhere onto the inner surface of the bag, chemical substances are not eluted from the inner surface of the bag during the blood platelet is stored in the bag, mechanical property of the bag is little changed at high or low temperatures, and the bag is excellent in flexibility and transparency.

A poly(ethylene-butylene)-polystyrene block copolymer used in the present invention comprises a hard segment and a soft segment. The hard segment is composed of a styrene polymer such as polystyrene or polymethylstyrene, and the soft segment is composed of a polyethylene-butylene copolymer. The polystyrene polymer used as a hard segment has a number average molecular weight of 5,000 to 120,000. The polyethylene-butylene copolymer used as a soft segment can be prepared by reacting polybutadiene having a number average molecular weight of 10,000 to 250,000 with polystyrene to give a poly(ethylene-butadiene)-polystyrene block copolymer, and thereafter hydrogenerating the block copolymer. The preferable molar ratio of ethylene and butylene contained in the polyethylene-butylene copolymer, which is a soft segment, is 40/60 to 60/40, and the preferable content of the soft segment in the block copolymer is 60 to 90 % by weight in order to give a block copolymer having excellent mechanical property and flexibility. It is preferable that the content of the block copolymer in the polymer mixture is 25 to 60 % by weight, more preferably 30 to 50 % by weight. When the content is less than 25 % by weight, there is a tendency that the flexibility of an obtained bag deteriorates, and when the content is more than 60 % by weight, there is a tendency that the moldability of the obtained bag deteriorates.

It is preferable that the number average molecular weight of the block copolymer is about 15,000 to 370,000, more preferably 30,000 to 250,000.

The terminology "polypropylene" used in the present invention is intended to refer to a homopolymer of propylene or a copolymer of which main component is propylene. Examples of the component other than propylene used in the copolymer are, for instance, ethylene, 1-butene, and the like. The content of the component other than propylene is preferably not more than 10 % by weight. The polypropylene has an ability to give a bag mechanical strength at the time of forming, and the mechanical strength of the bag is remarkably improved by mixing the polypropylene with the other polymer in a specific ratio when preparing a polymer alloy. The preferable content of the polypropylene in the polymer mixture is 20 to 40 % by weight, more preferably 25 to 35 % by weight. When the content is less than 20 % by weight, there is a tendency that the transparency and mechanical strength of an obtained bag deteriorate, and when the content is more than 40 % by weight, there is a tendency that the flexibility of an obtained bag deteriorates.

The ethylene-acrylic acid ester gives the block copolymer and polypropylene compatibility and contributes to the formation of a polymer alloy. Examples of the acrylic acid ester used in the present invention are, for instance, methyl acrylate, ethyl acrylate, butyl acrylate, and the like. The content of the acrylic acid ester in the copolymer is preferably 5 to 90 % by mole. When the content of acrylic acid ester is less than 5 % by mole, there is a tendency that transparency of the ethylene-acrylic acid ester copolymer deteriorates, and when the content of the acrylic acid ester is more than 90 % by mole, there is a tendency that the elastic modulus of the ethylene-acrylic acid ester copolymer at high temperatures deteriorates.

The content of the ethylene-acrylic acid ester copolymer in the polymer mixture is preferably 5 to 35 % by weight, more preferably 10 to 30 % by weight. When the content is less than 5 % by weight, there is a tendency that the dispersibility of the ethylene-acrylic acid ester copolymer in the polymer mixture and the workability at extruding deteriorate and the transparency of the polymer mixture is lowered. Further, there is a tendency that the physical properties at low temperatures deteriorate. When the content is more than 35 % by weight, there is a tendency that the mechanical strength of an obtained bag is lowered.

When the homogeneously mixed polymer mixture is subjected to the melt extrusion molding, the molecular chains of polystyrene segments which are hard segments of the block copolymer gather to form at least two oriented portions in the polymer mixture, and the polymer mixture forms a polymer alloy having a chemical constitution that the chains of a soft segment of the block copolymer, a hard segment of the block copolymer which is not oriented, polypropylene and ethylene-acrylic acid ester copolymer are entangled together to form a network structure.

The polymer mixture is formed into a polymer alloy having preferable properties required for a bag for storage of platelets, such as mechanical strength, in particular mechanical property at low temperatures with keeping flexibility and improved gas permeability such as oxygen permeability or carbon dioxide gas permeability.

After the above-mentioned three polymers are homogenously mixed together in a blender or a mixer, the polymer mixture is melted. The melted polymer mixture is extruded into a mold having a shape corresponding to a medical bag and is thereafter subjected to blow molding, or is extruded to give a sheet and is subsequently heat-sealed at the outer side of the sheet to give a bag having a prescribed shape. The thickness of the sheet can be suitably adjusted in accordance with the uses. The thickness is, for instance, 200 to 450 $\mu$m.

A multiple bag system of the present invention comprises a donor bag, one or more than one transfer bags and tubes. The number of the transfer bags is one or more than one as mentioned above. In particular, the number of transfer bags can be suitably adjusted to 1 to 3 as occasion demands.

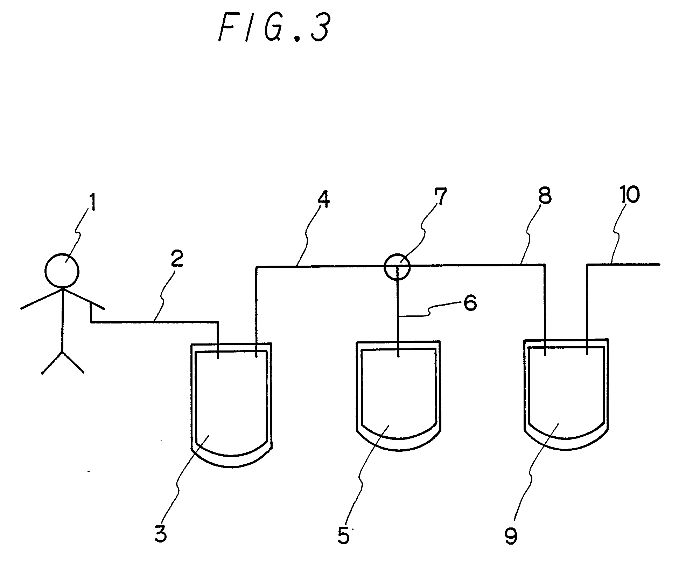

One embodyment of the bag for storage of platelets of the present invention, which is used as a transfer bag of a multiple bag system, is explained in accordance with Fig. 3.

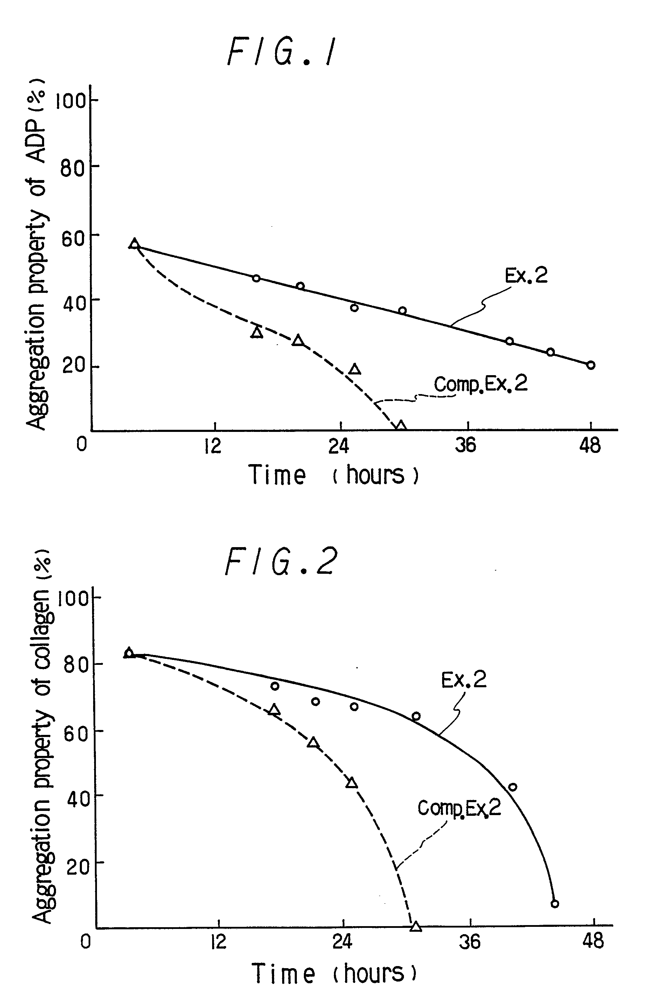

Fig. 3 is a drawing explaining an embodiment of a multiple bag. In Fig. 3, 1 denotes a donor, each of 2, 4, 6, 8 and 10 denotes a tube, 3 denotes a donor bag. 5 denotes a first transfer bag, 7 denotes a directional control valve, and 9 a second transfer bag.

Blood collected from the donor 1 is transferred into the donor bag 3. The blood is separated into blood corpuscle component and blood plasma component in the donor bag 3 by means of a centrifugal separator. It is preferably that a material of the donor bag 3 is a plastic material containing a plasticizer, and more preferably, polyvinyl chloride containing dioctyl phthalate since the supernatant contains less amount of hemoglobin during blood is stored in the donor bag 3. The content of the plasticizer in the plastic material can be selectively adjusted in accordance with the kind of the plasticizer and the combination of the plasticizer and the plastic material. The content is, for instance, 10 to 50 % by weight.

The blood plasma component separated from the blood in the donor bag 3 is transferred into the first transfer bag 5 through the tube 4 and the tube 6. The tubes 4 and the tube 6 are, for instance, made of polyvinyl chloride, or the like.

The first transfer bag 5 is made of the above-mentioned polymer alloy. Also, the blood components in the first transfer bag 5 are separated into blood platelet component and blood plasma component by means of a centrifugal separator, and the blood plasma component is transferred into the second transfer bag 9 through the tube 6 to the tube 8. The blood platelet is stored in the first transfer bag 5.

Examples of the material of the second transfer bag 9 are, for instance, the above-mentioned polymer alloy, polyvinyl chloride containing dioctyl phthalate, polyvinyl chloride containing 2-ethylhexyl trimellitate, and the like. The blood plasma component other than blood platelet, which is accomodated in the second transfer bag 9, is usually stored in a freezer.

The container for storage of platelet of the present invention is more specifically described and explained by means of the following Examples and Comparative Examples. However, it is to be understood that the present invention is not limited to these Examples, and various changes and modifications can be made in the invention without departing from the spirit and scope thereof.

Example 1

A mixture composed of 40 % by weight of Poly(ethylene-butylene)-polystyrene block copolymer (available from Shell Chemical Co., Ltd. under the trade name "KRATON G-1650"), 30 % by weight of polypropylene (available from Sumitomo Chemical Company, Limited under the trade name "FL-6711N") and 30 % by weight of ethylene-ethyl acrylate copolymer containing 15 % by mole of ethyl acrylate (available from Japan Uniker Co., Ltd. under the trade name "DPDJ 6182") was prepared by mixing these components with a mixer. The mixture was heated to a temperature of 200°C to melt, and was extruded to give a sheet having a thickness of 320 $\mu$m. With respect to the above sheet, hydrolysis test and measurement of dynamic modulus of elasticity were carried out. The results are shown in Table 1.

Comparative Example 1

A mixture was prepared in the same manner as in Example 1 except that the components of the mixture were changed into 40 % by weight of poly(ethylene-butylene)-polystyrene block copolymer, 30 % by weight of polypropylene and 30 % by weight of ethylene-vinyl acetate copolymer. The mixture was heated to a temperature of 200°C to melt, and was extruded to give a sheet having a thickness of 320 μm. With respect to the obtained sheet, hydrolysis test and measurement of dynamic modulus of elasticity were carried out in the same manner as in Example 1.

The results are shown in Table 1.

Table 1

| Ex. No. | Hydrolysis test (mg) | | Dynamic modulus of elasticity (kg/cm²) | | |
|---|---|---|---|---|---|
| | Ethanol *1 | Acetic acid *2 | -20°C | 20°C | 60°C |
| 1 | Not detected | Not detected | 4800 | 1430 | 610 |
| Com.Ex.No. | | | | | |
| 1 | Not detected | 763 | 5300 | 1510 | 585 |

*1: Detection limit is 3 ppm.
*2: Detection limit is 10 ppm.

Hydrolysis test and measurement of dynamic modulus of elasticity were carried out in accordance with the following methods.

(1) Hydrolysis test

The obtained sheet was cut to give test pieces. A pressure container was charged with 10 g of the test piece and 30 g of water, and the mixture was heated at 130°C for 2 hours. The degradation products contained in the water were detected by means of a gas chromatography.

(2) Dynamic modulus of elasticity

In accordance with ASTM D-638, dynamic modulus of the elasticity of the test pieces was measured by means of Vibron.

As is clear from Table 1, the sheet prepared in Example 1 has higher dynamic modulus of elasticity at high temperatures than that of the sheet prepared in Comparative Example 1, in which ethylene-vinyl acetate copolymer was used instead of ethylene-acrylic acid ester copolymer used in Example 1.

Also, the sheet prepared in Example 1 has lower dynamic modulus of elasticity at low temperatures than that of the sheet prepared in Comparative Example 1.

Accordingly, from the above results, it can be said that a bag composed of the sheet prepared in Example 1 is not softened and deformed at high temperatures and is not cured at low temperatures.

According to the hydrolysis test at high temperatures, acetic acid was detected from the sheet prepared in Comparative Example 1. However, from the sheet prepared in Example 1, neither ethanol nor acetic acid was detected.

Accordingly, it can be said that the sheet prepared in Example 1 can be suitably used as a material used in the bag for storage of platelets of the present invention because chemical substances are not eluted from the sheet, and the sheet can be subjected to high-pressure steam sterilization.

Example 2 and Comparative Example 2

A mixture composed of 54 % by weight of poly(ethylene-butylene)-polystyrene block copolymer (available from Shell Chemical Co., Ltd. under the trade name of "KRATON G-1650"), 23 % by weight of polypropylene (available from Sumitomo Chemical Company, Limited under the trade name of "FL-6711N") and 23 % by weight of ethylene-ethyl acrylate copolymer containing 15 % by mole of ethyl acrylate (available from Japan Uniker Co., Ltd. under the trade name of "DPDJ-6182") was prepared by mixing these components with a mixer.

The mixture was heated to a temperature of 200°C to melt and was extruded to give two sheets having a thickness of 250 μm. Then, a tubular port was disposed between the two sheets, and the two superposed sheets to the outside of the tubular port were heat-sealed to give a medical bag used in Example 2.

A bag for comparing with the above bag used in Example 2 was produced in the same manner as in Example 2 except that polyvinyl chloride containing 40 % by weight of dioctyl phthalate was used as a material for a bag, and that the polyvinyl chloride was heated to a temperature of 200°C to melt and the melted polyvinyl chloride was extruded to give a sheet having a thickness of 250 μm.

As the physical properties of the obtained bags, storage stability, gas permeability and storage stability were investigated in accordance with the following methods.

(Storage stability of blood platelet of rabbits)

The bag was charged with a concentrated solution of blood platelet prepared by centrifugating blood of rabbits at 1200 rpm for 10 minutes.

After the bag was subjected to horizontal vibration at 22°C for about two days, aggregation properties of adenosine diphosphate (hereinafter reffered to as "ADP") and collagen were measured. The results are shown in Figs. 1 and 2.

The measurement of aggregation properties of ADP and collagen was carried out by means of NKK Haemotoracer 1 (available from Niko Bioscience Co., Ltd.), which was a value at a time that the final concentration ADP was 40 μmol/mℓ or that of collagen was 10μg/mℓ as a reagent was added to the concentrated solution little by little.

The time when the value of aggregation property was half of the blank value was estimated from Figs. 1 and 2. As the results, according to ADP aggregation property, the time in Example 2 was about 36 hours and the time in Comparative Example 2 was about 18 hours, and according to collagen aggregation property, the time in Example 2 was about 38 hours and the time in Comparative Example 2 was about 26 hours.

Accordingly, as is clear from the above results, the bag of the present invention has ADP aggregation property of about 1.6 times longer than that of the bag obtained in Comparative Example 2, and collagen aggregation property of about 2.0 times longer than that of the bag obtained in Comparative Example 2.

(Gas permeability test of bag)

With respect to the bags obtained in Example 2 and Comparative Example 2, oxygen permeability, carbon dioxide gas permeability and steam permeability were measured in accordance with the following methods. The results are shown in Table 2.

(1) Oxygen permeability

Oxygen permeability of the bag was measured in accordance with ASTM-D-1434.

(2) Carbon dioxide gas permeability

Carbon dioxide gas permeability of the bag was measured in accordance with ASTM-D-1434.

(3) Steam permeability

Steam permeability of the bag was measured in accordance with JIS-Z-0208.

## Table 2

| Ex.No. | Oxygen permeability $(cc/m^2 \cdot atm \cdot 24hrs)$ | Carbon dioxide gas permeability $(cc/m^2 \cdot atm \cdot 24hrs)$ | Steam permeability $(g/m^2 \cdot 24hrs)$ |
|---|---|---|---|
| 2 | 1817 | 6645 | 6.0 |

| Ex.No. | Oxygen permeability $(cc/m^2 \cdot atm \cdot 24hrs)$ | Carbon dioxide gas permeability $(cc/m^2 \cdot atm \cdot 24hrs)$ | Steam permeability $(g/m^2 \cdot 24hrs)$ |
|---|---|---|---|
| Comp. Ex.No. | | | |
| 2 | 580 | 2694 | 21.0 |

As is clear from Table 2, the bag of the present invention has oxygen permeability of about 3.1 times larger than that of the bag obtained in Comparative Example 2, and carbon dioxide gas permeability of about 2.5 times larger than that of the bag obtained in Comparative Example 2, and steam permeability of about 1/3.5 of the steam permeability of the bag obtained in Comparative Example 2. Accordingly, the bag obtained in Example 2 can be preferably used as a bag for storage of platelets in comparison with the bag obtained in Comparative Example 2.

(Storage stability of human blood platelet)

The bag obtained in Example 2 was charged with a concentrated solution of human blood platelet prepared by means of a blood cell separator (available from Haemonetics Co., Ltd. under the trade name of "V-50"), and the blood platelet was stored in the bag while horizontally vibrating the bag at a temperature of 22°C.

The blood platelet was take out from the bag at intervals of 24 hours and aggregation properties of ADP and collagen were measured. The results are shown in Table 3.

In Table 3, each of ADP 10 and ADP 20 means the value of ADP aggregation property when the final concentration of ADP was 10 $\mu$mol/m$\ell$ and 20 $\mu$mol/m$\ell$, respectively, and each of CLG 5 and CLG 10 means the value of aggregation property when the final concentration of collagen was 5 $\mu$g/m$\ell$ and 10 $\mu$g/m$\ell$, respectively.

In Table 3, 5 days/0 day was evaluated by calculating the following equation.

[5 days/0 day] (%) = {(aggregation property on the 5th day)/(initial aggregation property)] x 100

As is clear from Table 3, the decrease of aggregation properties of ADP and collagen of the blood platelet in the bag obtained in Example 2 is lower than that in the bag obtained in Comparative Example 2 when 5 days passed. Accordingly, the bag of the present invention can be preferably used as a bag for storage of platelets in comparison with the bag obtained in Comparative Example 2.

Table 3

| Ex.No. | Reagent and final concentration thereof ($\mu$mol./m$\ell$) | Aggregation property of each day (%) | | | | | | 5 days/0 day |
|---|---|---|---|---|---|---|---|---|
| | | 0 day (Beginning) | 1 day | 2 days | 3 days | 4 days | 5 days | (%) |
| 2 | ADP 20 | 73.975 | 51.15 | 35.6 | 31.5 | 26.15 | 27.0 | 36.5 |
| Comp.Ex. 2 | ADP 20 | 73.975 | 30.625 | 22.3 | 15.75 | 16.75 | 11.61 | 15.7 |
| Ex. 2 | ADP 10 | 63.4 | 30.6 | 25.85 | 20.65 | 21.0 | 20.78 | 32.8 |
| Comp.Ex. 2 | ADP 10 | 63.4 | 18.3 | 13.95 | 10.3 | 10.9 | 7.56 | 11.9 |
| Ex. 2 | CLG 10 | 78.4 | 74.9 | 69.05 | 63.5 | 56.8 | 50.06 | 63.9 |
| Comp.Ex. 2 | CLG 10 | 78.4 | 65.95 | 57.3 | 43.85 | 40.9 | 31.5 | 40.2 |
| Ex. 2 | CLG 5 | 75.95 | 66.35 | 52.05 | 31.95 | 22.55 | 19.72 | 26.0 |
| Comp.Ex. 2 | CLG 5 | 75.95 | 50.4 | 34.6 | 20.65 | 12.8 | 7.33 | 10.0 |

## Examples 3 to 6 and Comparative Examples 3 and 4

A polymer mixture was prepared by mixing a block copolymer, polypropylene and ethylene-ethyl acrylate copolymer, which were the same components used in Example 1 in a mixing ratio shown in Table 4. The polymer mixture was heated to a temperature of 200°C to melt and was extruded to give a sheet having a thickness of 250 $\mu$m. With respect to the prepared sheets, mechanical properties and transparency were examined in accordance with the following methods. The results are shown in Table 4.

(1) Tensile strength

Tensile strength of the sheet was measured in accordance with JIS-Z-1702.

(2) Transparency

Transparency of the sheet was examined with the naked eyes on the basis of the following criteria.
○: Transparent
△: Slightly translucent
×: Translucent

8

Table 4

| Ex.No. | Components (% by weight) | | | Tensile strength (kg/cm²) | | | Transparency |
|---|---|---|---|---|---|---|---|
| | Block copolymer | Polypropylene | Ethylene-ethyl acrylate copolymer | 20°C | 60°C | 120°C | |
| 3 | 54 | 23 | 23 | 420 | 100 | 16 | ◯ |
| 4 | 25 | 40 | 35 | 350 | 110 | 15 | ◯ |
| 5 | 40 | 30 | 30 | 390 | 105 | 15 | ◯ |
| 6 | 60 | 35 | 5 | 405 | 112 | 18 | ◯ |
| Comp.Ex.No. | | | | | | | |
| 3 | 60 | 40 | 0 | 290 | 95 | 13 | × |
| 4 | 30 | 20 | 50 | 220 | 81 | 4 | △ |

As is clear from Table 4, the sheets obtained in Examples 3 to 6 are superior in tensile strength and transparency to the sheets obtained in Comparative Examples 3 and 4.

Example 7

With respect to the bag obtained in Example 2, a test for chemical extracts was carried out in accordance with following three methods. The results are shown in Table 5.

(A) Test for chemical extracts was carried out in accordance with the method regulated in Voluntary standards for plastic material for medical use prescribed by Japan association of disposable medical device industries.

(B) Test for chemical extracts was carried in accordance with testing methods for plastic container for transfusion regulated in Japanese pharmacopoeia.

(C) Test for chemical extracts was carried out in accordance with the standard for construction of blood bags made of polyvinyl chloride regulated in Notification No. 448 of the Ministry of Welfare, Japan.

## Table 5

| Ex.No. | Test method | Appearance | Foaming | pH | Chloride | Sulfate | Phosphate | Ammoniate |
|---|---|---|---|---|---|---|---|---|
| | A | Colorless and transparent | Suitable | 0.17 | — | — | — | — |
| 7 | B | Colorless and transparent | Suitable | 0.31 | Suitable | Suitable | Suitable | Suitable |
| | C | Colorless and transparent | — | 0.22 | Suitable | Suitable | — | Suitable |

EP 0 330 151 A2

- continued -

| Ex. No. | Test method | Tin | Heavy metal | Zinc (ppm) | Consumption of potassium permanganate (mℓ) | Evaporation residue (mg) | Ultraviolet absorption spectrum | Changes by heating |
|---|---|---|---|---|---|---|---|---|
| 7 | A | Not more than the limit | — | 0.22 | 0.1 | 0.3 | 0.01 | — |
|  | B | — | — | 0.00 | 0.2 | 0.5 | 0.02 | — |
|  | C | — | Suitable | — | 0.75 | 0.4 | — | Suitable |

EP 0 330 151 A2

As is clear from the above results, the bag of the present invention was acceptable for each of the methods A, B and C.

## Example 8

A donor bag made of polyvinyl chloride containing 35 % by weight of dioctyl phthalate was produced in the same manner as in Comparative Example 2.

The donor bag was used in a multiple bag shown in Fig. 3, and storage stability of blood stored in the donor bag was examined. The results are shown in Table 6.

## Example 9

A donor bag is produced in the same manner as in Example 8 except that the components used in the donor bag were changed into polyvinyl chloride containing 40 % by weight of 2-ethylhexyl trimellitate. The storage stability of blood stored in the obtained donor bag was examined in the same manner as in Example 8. The results are shown in Table 6.

## Comparative Example 5

A donor bag is produced in the same manner as in Example 8 except that the components used in the donor bag were changed into the polymer alloy used in Example 1. The storage stability of blood stored in the obtained donor bag was examined in the same manner as in Example 8. The results are shown in Table 6.

Table 6

| Ex. No. | Content of ATP ($\mu$mol./gHb) | Content of 2,3-DPG ($\mu$mol./gHb) | Content of hemoglobin in supernatant (mg/d$\ell$) | pH | $P_{CO_2}$ (mmHg) |
|---|---|---|---|---|---|
| (Blank) | 4.5 | 16.4 | 2.6 | 7.0 | 67.8 |
| 8 | 2.8 | 2.7 | 41.5 | 6.7 | 105.4 |
| 9 | 3.0 | 2.9 | 63.7 | 6.8 | 97.2 |
| Comp.Ex. | | | | | |
| 5 | 3.2 | 3.0 | 62.0 | 6.9 | 53.0 |

The physical properties shown in Table 6 were examined by taking out 0.2 cc of blood from the donor bag before the blood was stored in a freezer or after the blood was stored in a freezer for 3 weeks.

The contents of adenosine triphosphate (ATP) and 2,3-diphenylguanidine (2,3-DPG) in the blood, and the content of hemoglobin in the supernatant of the blood were measured in accordance with the following methods. Also, the partial pressure of carbon dioxide gas was examined by taking out 0.5 cc of blood from the donor bag.

(1) Content of ATP ($\mu$mol./gHb)

The content of ATP contained in the blood was measured in accordance with the enzymic method of the ATP measuring kit (available from Behringer Co., Ltd.).

(2) Content of 2,3-DPG ($\mu$mol./gHb

The content of 2,3-DPG contained in the blood was measured in accordance with the enzymic method of the DPG measuring kit (available from Behringer Co., Ltd.).

(3) Content of hemoglobin in the supernatant of the blood (mg/d$\ell$)

The content of hemoglobin contained in the supernatant of the blood was measured in accordance with the azid-hemoglobin method by means of a hemoglobin measuring kit (available from NIHON SHOJI Co., Ltd.).

(4) Partial pressure of carbon dioxide gas ($P_{CO_2}$)

The partial pressure of carbon dioxide gas in the blood was measured by injecting 0.5 cc of the blood into Radiometer IBL3 (available from Radiometer Co., Ltd.).

As is clear from Table 6, the partial pressure of carbon dioxide gas of the blood stored in the donor bags prepared in Examples 8 and 9, which were made of polyvinyl chloride containing a plasticizer, is higher than that of the blood stored in the donor bag prepared in Comparative Example 5. Also, the contents of ATP and 2,3-DPG contained in the blood stored in the donor bags which were prepared in Examples 8 and 9 are lower than those of the blood stored in the donor bag prepared in Comparative Example 5.

In particularly, the bag prepared in Example 8 which was made of polyvinyl chloride containing dioctyl phthalate which was used as a plasticizer can be preferably used since the content of hemoglobin contained in the supernatant of the blood was low.

As mentioned above, blood platelet can be stored in a bag made of the polymer alloy of the present invention safely for a long period of time because there are occurred little adherence and aggregation of the blood platelet on the inner surface of the bag, and there are no depression of the blood platelet such as hypotonic shock response during the blood platelet is stored in the bag made of the polymer alloy.

The bag of the present invention can be also preferably stored in a freezer having a temperature of not more than -20° C because the bag possesses flexibility and preferable mechanical properties at such a low temperature. Further, the bag of the present invention can be subjected to high-pressure steam sterilization since the bag is not softened and transformed at high temperatures and does not generate decomposition products by heat decomposition or hydrolysis of the bag.

Accordingly, it can be said that the bag of the present invention can be preferably used as a bag for storage of platelets since the bag has preferable oxygen permeability and carbon dioxide gas permeability.

Also, the components which are separated from blood collected from a donor can be preferably stored for a long period of time in the bag for storage of platelets of the present invention, and the bag can be preferably used as a transfer bag of a multiple bag system.

Having described a specific embodiment of our bearing, it is believed obvious that modification and variation of our invention is possible in light of the above teachings.

## Claims

1. A bag for storage of platelets comprising a polymer alloy consisting essentially of
   (a) 25 to 60 % by weight of poly(ethylene-butylene)-polystyrene blcok copolymer,
   (b) 20 to 40 % by weight of polypropylene, and
   (c) 5 to 35 % by weight of ethylene-acrylic acid ester copolymer, the segment of said polystyrene in the molecular chains of said polymer (a) having at least 2 oriented portions and the molecular chains of said polymers (a), (b) and (c) being entangled to form a network structure.

2. A multiple bag system comprising
   (a) a donor bag for receiving blood from a donor and separating the components of the blood,
   (b) one or more than one transfer bags for storing the separated blood components, of which material is different from that of the donor bag, and

(c) tubes for connecting the donor bag with the transfer bag and transferring the separated blood components from the donor bag to the transfer bag, wherein said transfer bag comprises a polymer alloy consisting essentially of
(i) 25 to 60 % by weight of poly(ethylene-butylene)-polystyrene block copolymer,
(ii) 20 to 40 % by weight of polypropylene, and
(iii) 5 to 35 % by weight of ethylene-acrylic acid ester copolymer, the segment of said polystyrene in the molecular chains of said polymer (a) having at least 2 oriented portions and the molecular chains of said polymers (i), (ii) and (iii) being entangled to form a network structure.

3. The multiple bag system of Claim 2, wherein said donor bag comprises polyvinyl chloride containing dioctyl phthalate.

# F I G . 1

# F I G . 2

# FIG.3